(19) **Europäisches Patentamt European Patent Office Office européen des brevets**

(11) **EP 0 959 350 B1**

(12) **FASCICULE DE BREVET EUROPEEN**

(45) Date de publication et mention
de la délivrance du brevet:
**15.09.2010 Bulletin 2010/37**

(51) Int Cl.:
***G01N 29/26*** *(2006.01)*    ***G10K 11/34*** *(2006.01)*

(21) Numéro de dépôt: **99401106.2**

(22) Date de dépôt: **06.05.1999**

(54) **Procédé de contrôle ultrasonore de pièces à géométrie cylindrique**

Ultraschallprüfverfahren für zylindrische Werkstücke

Process for ultrasonic testing of cylindrical pieces

(84) Etats contractants désignés:
**BE DE ES FR GB IT SE**

(30) Priorité: **07.05.1998 FR 9805776**

(43) Date de publication de la demande:
**24.11.1999 Bulletin 1999/47**

(73) Titulaire: **SOCIETE NATIONALE D'ETUDE ET DE CONSTRUCTION DE MOTEURS D'AVIATION "SNECMA" 75015 Paris (FR)**

(72) Inventeurs:
• **Beffy, Lionel**
**91080 Courcouronnes (FR)**
• **Fink, Alexandre Mathias**
**92190 Meudon (FR)**
• **Mangenet, Gérard Yves**
**91860 Epinay sous Senart (FR)**
• **Miette, Véronique**
**75011 Paris (FR)**
• **Wu, François Jean**
**91400 Orsay (FR)**

(74) Mandataire: **Joly, Jean-Jacques et al Cabinet Beau de Loménie 158, rue de l'Université 75340 Paris cedex 07 (FR)**

(56) Documents cités:
**EP-A- 0 480 086    EP-A- 0 598 661
EP-A- 0 898 175    WO-A-96/24053
WO-A-98/34294    US-A- 5 457 996**

## Description

L'invention concerne un procédé de contrôle ultrasonore en immersion de pièces à géométrie cylindrique. Elle s'applique en particulier à la détection de défauts internes dans le volume de billettes telles que des billettes de titane dans des applications aéronautiques. Il existe de nombreux dispositifs de contrôle de pièce par ultrasons fonctionnant en transmission ou en réflexion. Le contrôle ultrasonore de pièces peut être effectué en utilisant une pluralité de transducteurs acoustiques élémentaires à focalisation fixe pour détecter des défauts à différentes profondeurs de la pièce. Chaque transducteur émet un faisceau ultrasonore focalisé à une profondeur prédéterminée de la pièce à contrôler. Les transducteurs ont des zones focales situées à des profondeurs croissantes permettant de contrôler le volume complet, de la surface jusqu'au centre de la pièce cylindrique.

Le contrôle ultrasonore de pièces peut également être effectué en utilisant une sonde acoustique comportant plusieurs éléments transducteurs à focalisation électronique, la focalisation étant obtenue par application de différents temps de retards aux signaux délivrés par chaque transducteur. Pour réaliser une image en profondeur de la pièce en utilisant comme source ou comme récepteur un réseau de transducteurs, une difficulté majeure provient du fait que les échos dûs à la réflexion sur la microstructure du matériau sont élevés par rapport aux échos éventuels provenant des défauts et que ces échos masquent les échos de défauts à identifier. Le problème est encore aggravé lorsque la pièce a une forme complexe et/ou une structure hétérogène comme c'est le cas notamment des billettes de titane.

Pour résoudre ce problème, il est connu d'utiliser une technique qualifiée d'amplification ultrasonore à conjugaison de phase, appelée aussi retournement temporel. Ce procédé consiste, après émission d'un faisceau ultrasonore non ou peu focalisé et réception de l'écho renvoyé par le défaut à localiser, à réémettre ce signal d'écho après retournement temporel de sa répartition dans le temps et de sa forme. Une application de ce procédé au contrôle ultrasonore de pièces de révolution est décrit dans le document FR 2 698 170. Ce procédé permet de réaliser une focalisation optimale sur des éventuels défauts dans des pièces à structure hétérogène telles que des pièces en titane, mais présente l'inconvénient majeur d'exiger beaucoup de temps pour réaliser la focalisation du faisceau ultrasonore, ce qui augmente de façon importante le temps de contrôle des pièces par rapport aux procédés classiques utilisant la focalisation électronique.

Le but de l'invention est de pallier les inconvénients des procédés de contrôle ultrasonore connus et de réaliser un procédé de contrôle ultrasonore en immersion permettant de contrôler des pièces à géométrie et à structure interne complexe telles que des billettes de titane, le contrôle du volume de la pièce étant effectué dans un minimum de temps avec un niveau de sensibilité de détection constant et le plus élevé possible.

Pour cela, le procédé de contrôle selon l'invention consiste à utiliser un transducteur multi-éléments émettant des ondes ultrasonores focalisées dans la pièce, la focalisation étant réalisée selon deux modes différents au moyen d'un dispositif électronique de commande multivoies, les deux modes de focalisation, appelés respectivement mode de focalisation électronique et mode de focalisation par retournement temporel, étant sélectionnés en fonction de la profondeur de la zone d'analyse considérée.

Le mode de focalisation par retournement temporel est choisi pour contrôler des zones centrales localisées aux profondeurs les plus importantes situées autour de l'axe longitudinal de la pièce cylindrique.

Le mode focalisation électronique est choisi pour contrôler des zones intermédiaires localisées à des profondeurs situées entre les zones centrales et une zone périphérique de la pièce cylindrique.

Selon l'invention le procédé de contrôle ultrasonore d'une pièce à géométrie cylindrique est conforme à la revendication 1.

D'autres particularités et avantages de l'invention apparaîtront clairement dans la suite de la description donnée à titre d'exemple non limitatif et faite en regard des figures annexées qui représentent :

- la figure 1, une vue simplifiée, en coupe longitudinale d'un dispositif de contrôle ultrasonore, selon l'invention ;

- la figure 2, une vue en coupe transversale partielle d'une tranche de billette à contrôler, selon l'invention ;

- la figure 3, un exemple de tache focale d'un transducteur focalisant à l'intérieur d'une pièce ;

- la figure 4, un exemple de chronogramme des émissions d'ondes ultrasonores dans différentes zones de la pièce et pour une tranche de billette,

- la figure 5, un schéma de principe d'une voie de traitement associée & un élément du transducteur multi-éléments dans un dispositif électronique multivoies, selon l'invention.

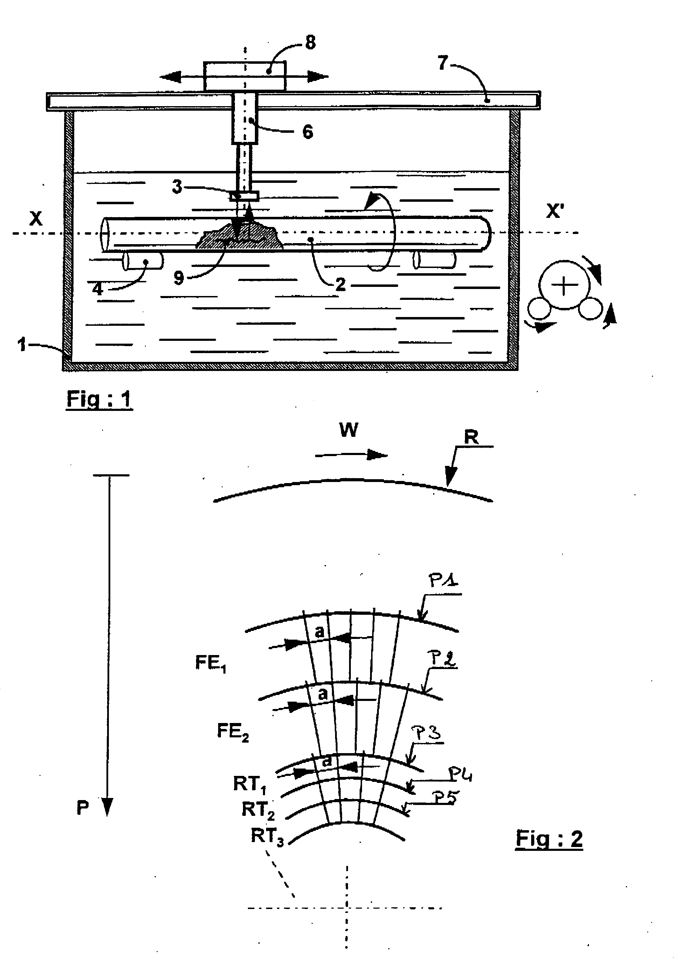

La figure 1 montre, en coupe longitudinale, le dispositif de contrôle par ultrasons d'une pièce de révolution, par exemple une billette de titane, selon l'invention.

Ce dispositif comprend une cuve 1 pleine d'un liquide de couplage acoustique dont le niveau de la surface est maintenu constant grâce, par exemple, à un système de trop-plein (non représenté). Dans un souci de simplification, les canalisations de remplissage et de vidange ont été omises. A l'intérieur de la cuve 1 sont plongés une pièce à contrôler 2 et un transducteur à ultrasons 3. Des rouleaux d'entrainement 4 assurent un centrage rigoureux de la pièce de révolution 2 selon son axe XX'. Le transducteur 3 est maintenu rigoureusement perpendiculaire à l'axe XX' par un support 6 qui est par ailleurs réglable, notamment en hauteur. En outre, et de façon connue, le dispositif comporte des moyens, non représentés, pour entraîner en rotation la pièce 2 avec une vitesse angulaire déterminée et des moyens (7, 8) pour déplacer longitudinalement le transducteur.

Le transducteur 3 est un transducteur émetteur/récepteur multi-éléments constitué de pastilles piézo-électriques organisées selon une matrice comme il est décrit plus avant.

L'utilisation de la cuve d'immersion 1 présente de nombreux avantages et, notamment, évite de mettre en contact le transducteur avec la pièce à contrôler, le couplage étant assuré par de l'eau additionnée éventuellement de produits mouillants tels que de l'huile. Toutefois, cette immersion totale n'est pas obligatoire dans le cadre de la présente invention et le contrôle peut être assuré en plaçant simplement le transducteur contre la pièce à contrôler, au moyen d'un mince film de couplant.

Le principe de mesure utilisé repose sur l'émission d'un train d'ondes ultrasonores E qui pénètre dans la pièce à contrôler 2 et est en partie réfléchi R en en présence d'un défaut 9 dans l'épaisseur de la pièce. La fréquence d'émission des trains d'ondes successifs est appelée fréquence de récurrence de la mesure. Le contrôle de la pièce de révolution 2 est réalisé en continu, la pièce étant en rotation.

Le contrôle est réalisé par tranches successives par déplacement longitudinal, pas à pas, du transducteur 3. La figure 2 représente une vue en coupe transversale partielle d'une tranche de billette à contrôler.

Chaque tranche de billette est divisée en couronnes annulaires situées à des profondeurs de billette différentes, chaque couronne annulaire étant elle-même divisée en secteurs angulaires successifs régulièrement répartis. Chaque secteur angulaire constitue une zone d'analyse. Sur la figure 2, six couronnes sont représentées. La première couronne correspond à une zone périphérique de la billette, contrôlée de manière connue par un ou plusieurs transducteurs mono éléments de petites dimensions focalisés chacun à une profondeur donnée.

Les deux couronnes suivantes correspondent à des zones intermédiaires FE1 et FE2 de profondeur moyenne P1, respectivement P2. Les trois dernières couronnes correspondent aux zones les plus profondes RT1, RT2, RT3 de profondeurs respectives P3, P4, P5.

Le procédé de contrôle ultrasonore de la billette consiste à émettre des ondes ultrasonores focalisées dans la pièce, la focalisation étant réalisée selon deux modes différents sélectionnés en fonction de la profondeur de la zone de la pièce à contrôler.

Pour contrôler les zones FE1 et FE2 situées à des profondeurs intermédiaires P1, P2, le mode de focalisation électronique est utilisé. Pour contrôler les zones plus profondes RT1, RT2, RT3, le mode de focalisation par retournement temporel est utilisé.

Dans le mode focalisation électronique, la focalisation est réalisée par l'application de retards temporels prédéterminés à chacune des pastilles piézo-électriques constituant le transducteur. Le transducteur est piloté électroniquement, les retards étant déterminés avant le lancement de l'inspection, par exemple au moyen d'un logiciel de modélisation. Les retards peuvent également être acquis par apprentissage lors d'un étalonnage par autofocalisation, l'autofocalisation pouvant être réalisée dans un mode auto focus, ou dans un mode retournement temporel, appliqué sur une pièce étalon contenant des défauts de référence afin de déterminer les meilleures lois de retards à mettre en oeuvre sur la billette à contrôler. La loi de retard peut aussi être modulée en amplitude selon les voies (une voie étant associée à une pastille piézo-électrique).

Le mode autofocus et le mode retournement temporel sont des procédés qui réalisent une autofocalisation sur un défaut qui présente une impédance acoustique différente de celle du milieu environnant. L'étalonnage réalisé dans un mode autofocus, consiste à émettre un faisceau d'ondes ultrasonores non focalisé dirigé vers une zone d'analyse de la pièce étalon et à déterminer les maxima des signaux d'écho reçus sur chaque voie afin d'en déduire les retards temporels liés au défaut réflecteur de référence considéré. La loi de retard ainsi définie n'est valable que pour la profondeur du réflecteur étalon et dans la limite de la profondeur de champ de la tache focale. Il est nécessaire de réaliser la même opération sur des réflecteurs étalons situés à des profondeurs différentes couvrant toute l'épaisseur de la pièce à contrôler. L'ensemble des lois est mémorisé et utilisé dans la phase d'inspection de la pièce.

L'étalonnage réalisé dans un mode retournement temporel comporte plusieurs étapes. La première étape consiste à émettre un faisceau d'ondes ultrasonores non ou peu focalisé dirigé vers une première zone d'analyse de la pièce étalon. La deuxième étape consiste à recevoir des premiers signaux d'écho renvoyés par un défaut de référence et le milieu environnant et à mémoriser sur chaque pastille piézo-électrique, la forme et la position dans le temps des signaux d'écho ; dans une troisième étape, à réémettre ces signaux dans la même zone d'analyse, dans une chronologie inverse, le dernier signal reçu étant le premier signal réémis. Il est possible de réitérer les deuxième et troisième étapes afin que les échos du défaut de plus forte réflectivité soient amplifiés par rapport à ceux du milieu environnant. Les maxima des signaux correspondant au défaut réflecteur de référence détecté sont ensuite déterminés et les retards temporels liés au défaut de référence sont déduits. De même que pour le mode autofocus, il est nécessaire de réaliser la même opération sur des défauts réflecteurs étalons situés à des profondeurs différentes couvrant toute l'épaisseur de la pièce à contrôler. L'ensemble des lois obtenues pendant cette phase d'apprentissage est mémorisé et utilisé dans la phase d'inspection de la pièce.

Le contrôle des zones les plus profondes de la billette est effectué intégralement dans un mode retournement temporel. Le procédé de contrôle décrit ci-dessus pour réaliser un étalonnage est utilisé sur la billette, en remplaçant la pièce étalon par la billette ; ou alors l'ensemble des lois obtenues sur étalon par apprentissage est utilisé en tant que 1er tir sur la billette de la séquence de retournement temporel.

Chaque émission d'ondes ultrasonores dans le mode focalisation électronique, ou chaque séquence de trois émissions successives dans le mode retournement temporel, est focalisée dans un secteur prédéterminé de la billette, le point de focalisation étant entouré d'une tache focale allongée selon l'axe de propagation des ondes ultrasonores et ayant une forme cylindrique dans sa partie médiane.

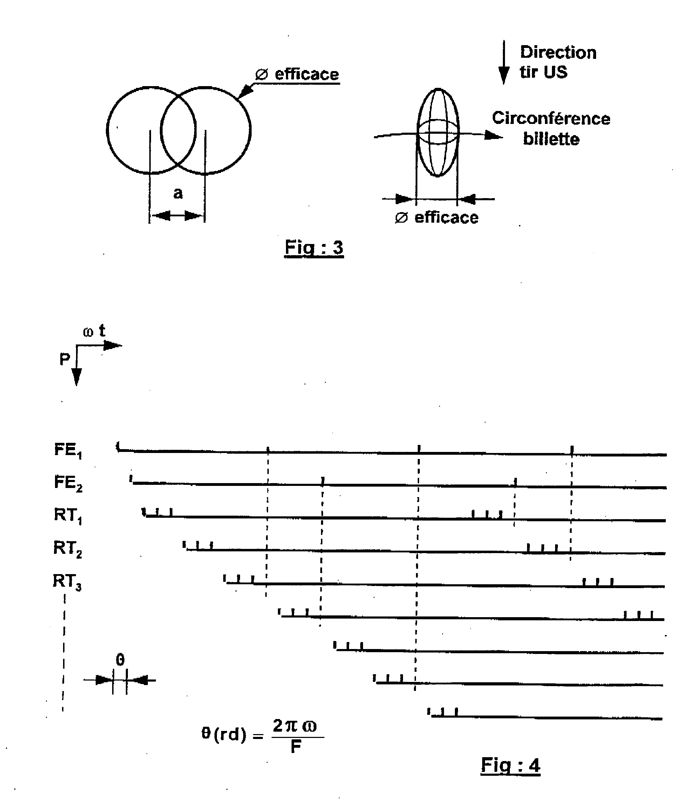

Un exemple de tache focale est représentée sur la figure 3.

Le pas d'avance, noté a, de la billette entre deux secteurs voisins dans la direction circonférentielle est inférieur au diamètre efficace, noté Ø efficace, de la tache focale de façon qu'il y ait un recouvrement transversal des taches focales correspondant à l'illumination des deux secteurs voisins. Le diamètre efficace de la tache focale est défini comme le plus grand diamètre de la tache dans sa partie cylindrique médiane et selon un plan médian perpendiculaire à l'axe de propagation des ondes ultrasonores.

Par exemple, le pas d'avance peut être choisi égal à 70 % du diamètre de la tache focale.

Afin de maintenir le même niveau de sensibilité de détection des défauts dans tout le volume inspecté, le pas d'avance de la billette entre deux émissions ou deux séquences d'émission dans deux secteurs voisins, est à peu près constant quelle que soit la profondeur inspectée.

Le secteur angulaire $\alpha_p$ correspondant dépend de la profondeur

$$P : \alpha_p = \frac{a}{2\pi\ (R-P)}$$

R étant le rayon de la billette. Le secteur angulaire augmente quand la profondeur augmente, ce qui signifie que le nombre d'émissions ultrasonores nécessaires diminue lorsque la profondeur contrôlée augmente.

La figure 4 représente un exemple de chronogramme des émissions d'ondes ultrasonores dans les différentes zones de la billette et pour une tranche de billette.

Une tranche de billette est examinée pendant un seul tour de rotation de la billette. Il faut donc, qu'en un tour de rotation l'ensemble des émissions d'ondes ultrasonores nécessaires au contrôle du volume correspondant à une tranche de billette, ait été réalisé.

Sur un tour de billette la focalisation des ondes émises est effectuée selon le mode focalisation électronique FE1, FE2 ou selon le mode retournement temporel RT1, RT2, RT3 suivant la profondeur des zones à contrôler. Ces deux modes sont utilisés de manière entrelacée afin de couvrir de façon optimale l'ensemble du volume de la tranche de billette à contrôler.

Cette optimisation est effectuée d'une part, en tenant compte du fait que plusieurs émissions successives sont nécessaires dans le mode retournement temporel et d'autre part, en tenant compte du pas d'avance de la billette dans la direction circonférentielle entre deux émissions, ou entre deux séquences d'émissions, successives.

Le nombre n d'émissions ultrasonores sur un tour de billette est lié à la vitesse de rotation ω de la billette et à la fréquence de récurrence F des tirs :

$$n = \frac{F\ (Hertz)}{\omega\ (nombre\ de\ tours/\ seconde)}$$

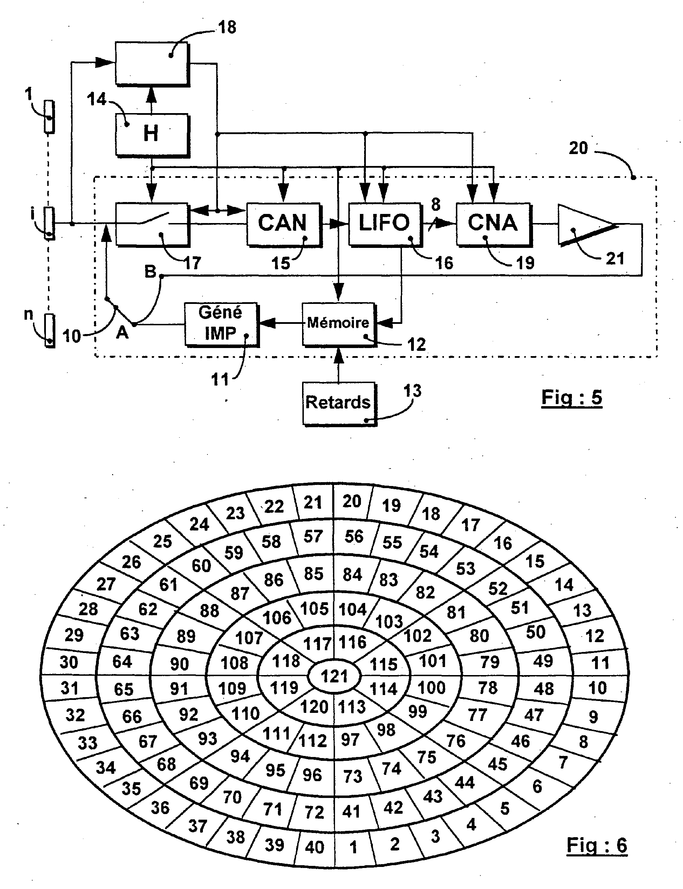

Le mode retournement temporel nécessitant plusieurs émissions successives (le plus souvent trois émissions), il est nécessaire que le déplacement de la zone contrôlée entre chaque émission soit limité. A ce déplacement limité correspond un secteur angulaire $\theta = \frac{2\pi\ \omega}{F}$ La figure 5 représente un schéma de principe d'une voie de traitement associée à un élément d'ordre i du transducteur dans un dispositif électronique multi-voies, selon l'invention.

Chaque voie comporte un commutateur 10 permettant de sélectionner l'une des deux modes de fonctionnement possibles. La position A sélectionne le mode focalisation électronique, la position B sélectionne le mode retournement temporel.

Dans le mode focalisation électronique, l'élément d'ordre i du transducteur reçoit des impulsions d'un générateur d'impulsions 11. Le retard appliqué aux impulsions émises est délivré par une mémoire 12, la mémoire 12 étant pilotée par une horloge interne 14. Le contenu de la mémoire 12 peut être géré de deux façons différentes selon que la loi de retard est déterminée de façon théorique par un logiciel de modélisation, dans ce cas les retards sont stockés dans un fichier 13, ou que la loi de retard est déterminée par auto focalisation sur des défauts d'une pièce étalon, dans ce cas les retards sont acquis par apprentissage, numérisé par un convertisseur analogique/numérique 15 et stockés dans une mémoire 16.

Dans le mode retournement temporel, chaque voie comporte un échantillonneur 17 destiné à fournir des échantillons analogiques du signal reçu par le transducteur i à la fréquence de l'horloge 14 pendant des intervalles de temps fixés par un cadenceur 18 et de durée T suffisante pour que l'écho soit reçu par tous les éléments du transducteur. L'échantillonneur 17 est suivi du convertisseur analogique/numérique 15. Il suffit en général d'une conversion sur huit bits pour représenter de façon satisfaisante la dynamique des échos. Les octets représentatifs chacun d'un échantillon sont mémorisés dans la mémoire 16 organisée en file d'attente (du type dernier entré-premier sorti) de capacité suffisante pour stocker tous les échantillons reçus pendant la durée T : le retournement temporel n'est effectué que sur cette durée T.

Le cadenceur 18 est prévu de façon à provoquer le début de l'échantillonnage au bout d'un temps déterminé après excitation par le générateur d'impulsions 11, une telle estimation étant facile à effectuer à partir de la connaissance de la vitesse de propagation des ultrasons dans le milieu.

Le cadenceur 18 est également prévu de façon à provoquer l'émission du front d'onde retourné au bout d'un temps bref après la fin de l'écho. Il est en effet souhaitable que ce temps soit bref (quelques milli-secondes par exemple) pour que ni le milieu, ni la position de l'objet ne se soient modifiés entre l'aller et le retour.

Pour permettre l'émission d'un front d'onde retourné, chaque voie 20 comprend un convertisseur numérique/analogique 19 suivi d'un amplificateur 21 de gain élevé. La sortie de l'amplificateur 21 attaque le transducteur respectif i.

Le figure 6 représente un exemple de transducteur multi-éléments, selon l'invention.

les éléments du transducteur numérotés de 1 à 121 sont des pastilles piézo-électriques organisées en matrice. Le pavage de la matrice est annulaire et sectoriel. Les 121 éléments ont sensiblement les mêmes dimensions. Le transducteur est préformé de manière à être préfocalisé dans la zone du coeur de la billette, la préfocalisation est assurée par une surface de Fermat.

## Revendications

1. Procédé de contrôle ultrasonore d'une pièce à géométrie cylindrique ayant un axe central longitudinal, qui consiste, pendant la rotation de la pièce, à émettre des ondes ultrasonores focalisées dans des zones à analyser situées à différentes profondeurs de la pièce, la focalisation étant réalisée avec un même transducteur multi-éléments selon deux modes de focalisation différents, un mode de focalisation par retournement temporel étant sélectionné pour contrôler des zones centrales situées au coeur de la pièce cylindrique autour de l'axe central longitudinal et un mode de focalisation électronique, par une application de retards temporels prédéterminés sur différentes voies d'émission des ondes ultrasonores, étant sélectionné pour contrôler des zones intermédiaires situées entre les zones centrales et des zones périphériques de la pièce cylindrique, les deux modes de focalisation étant appliqués de manière entrelacée dans le temps afin de couvrir de façon optimale l'ensemble du volume de la pièce à contrôler.

2. Procédé selon la revendication 1, **caractérisé en ce que** les retards temporels sont prédéterminés au moyen d'un logiciel de modélisation.

3. Procédé selon la revendication 2, **caractérisé en ce que** les retards temporels sont prédéterminés lors d'un étalonnage par un procédé d'autofocalisation appliqué sur une pièce étalon comportant des défauts de référence localisée en différentes profondeurs de la pièce étalon.

4. Procédé selon la revendication 3, **caractérisé en ce que** le procédé d'autofocalisation consiste à émettre un faisceau d'ondes ultrasonores non focalisé vers une zone d'analyse de la pièce étalon, à recevoir des échos réfléchis par des défauts de référence, à détecter des maxima des échos reçus et des retards temporels associés aux maxima des échos reçus, et à mémoriser ces retards temporels.

5. Procédé selon la revendication 3, **caractérisé en ce que** le procédé d'autofocalisation est un procédé de focalisation par retournement temporel appliqué sur une pièce étalon comportant des défauts de référence et nécessite plusieurs itérations d'émission et de réception de signaux ultrasonores dans chaque zone d'analyse.

6. Procédé selon l'une quelconque des revendications précédentes, **caractérisé en ce que** la pièce est analysée par tranches successives, chaque tranche étant divisée en couronnes annulaires et chaque couronne étant divisée en secteurs angulaires, chaque secteur angulaire constituant une zone d'analyse.

7. Procédé selon la revendication 6, **caractérisé en ce que** chaque tranche est analysée pendant un tour de rotation de la pièce.

8. Procédé selon la revendication 6, **caractérisé en ce que** l'analyse de deux secteurs successifs est réalisée par un déplacement circonférentiel de la pièce d'un pas d'avance prédéterminé et à peu près constant quelle que soit la profondeur de la zone analysée.

## Claims

1. A method for ultrasonic inspection of a part with a cylindrical geometry having a longitudinal central axis, which consists, during the rotation of the part, of emitting ultrasonic waves focused in areas to be analyzed located at different depths of the part, focusing being achieved with a same multi-element transducer according to two different focusing modes, a focusing mode with time reversal being selected for inspecting central areas located in the core of the cylindrical part around the longitudinal axis and an electronic focusing mode by applying predetermined time delays on different emission paths of ultrasonic waves being selected for inspecting intermediate areas located between the central areas and peripheral areas of the cylindrical part, both focusing modes being applied in a time-interlaced way in order to optimally cover the whole of the volume of the part to be inspected.

2. The method according to claim 1, **characterized in that** the time delays are predetermined by means of a modeling software package.

3. The method according to claim 2, **characterized in that** the time delays are predetermined during calibration by a self-focusing method applied on a standard part including reference defects localized at different depths of the standard part.

4. The method according to claim 3, **characterized in that** the self-focusing method consists of emitting a non-focused beam of ultrasonic waves towards an analysis area of the standard part, receiving echoes reflected by reference defects, detecting maxima of the received echoes and time delays associated with the maxima of the received echoes, and storing these time delays in memory.

5. The method according to claim 3, **characterized in that** the self-focusing method is a focusing method with time reversal applied on a standard part including reference defects and requires several iterations of emission and reception of ultrasonic signals in each analysis area.

6. The method according to any of the preceding claims, **characterized in that** the part is analyzed per successive slices, each slice being divided into annular crowns and each crown being divided into angular sectors, each angular sector forming an analysis area.

7. The method according to claim 6, **characterized in that** each slice is analyzed during one rotational turn of the part.

8. The method according to claim 6, **characterized in that** the analysis of two successive sectors is performed by circumferential displacement of the part by one predetermined advance step and approximately constant regardless of the depth of the analyzed area.

**Patentansprüche**

1. Verfahren zur Ultraschallprüfung eines Teils mit zylindrischer Geometrie und einer mittleren Längsachse, das darin besteht, während der Drehens des Teils gebündelte Ultraschallwellen in zu analysierende Bereiche, die in unterschiedlichen Tiefen des Teils gelegen sind, zu senden, wobei die Bündelung mit einem gleichen Multielementwandler nach zwei unterschiedlichen Bündelungsarten vollzogen wird, wobei eine Art der Bündelung durch zeitliche Umkehr gewählt wird, um zentrale Bereiche, die im Innersten des zylindrischen Teils um die mittlere Längsachse herum gelegen sind, zu prüfen, und eine Art der elektronischen Bündelung durch ein Anlegen vorbestimmter zeitlicher Verzögerungen an unterschiedlichen Sendewegen der Ultraschallwellen gewählt wird, um Zwischenbereiche, die zwischen den zentralen Bereichen und Randbereichen des zylindrischen Teils gelegen sind, zu prüfen, wobei die beiden Bündelungsarten zeitlich verschränkt angewandt werden, um das gesamte Volumen des zu prüfenden Teils optimal abzudecken.

2. Verfahren nach Anspruch 1, **dadurch gekennzeichnet, daß** die zeitlichen Verzögerungen mittels einer Modellierungssoftware vorbestimmt werden.

3. Verfahren nach Anspruch 2, **dadurch gekennzeichnet, daß** die zeitlichen Verzögerungen während eines Eichens durch ein Selbstfokussierungsverfahren, das auf ein Musterstück angewandt wird, welches in unterschiedlichen Tiefen des Musterstücks befindliche Referenzfehler aufweist, vorbestimmt werden.

4. Verfahren nach Anspruch 3, **dadurch gekennzeichnet, daß** das Selbstfokussierungsverfahren darin besteht, einen nicht gebündelten Strahl von Ultraschallwellen in Richtung eines Analysebereichs des Musterstücks auszusenden, durch Referenzfehler reflektierte Echos zu empfangen, Maxima der empfangenen Echos sowie den Maxima der empfangenen Echos zugeordnete zeitliche Verzögerungen zu erfassen und diese zeitlichen Verzögerungen zu speichern.

5. Verfahren nach Anspruch 3, **dadurch gekennzeichnet, daß** das Selbstfokussierungsverfahren ein Bündelungsverfahren durch zeitliche Umkehr, angewandt auf ein Referenzfehler aufweisendes Musterstück, ist und mehrere Wiederholungen des Sendens und Empfangens von Ultraschallsignalen in jedem Analysebereich erfordert.

6. Verfahren nach einem der vorhergehenden Ansprüche, **dadurch gekennzeichnet, daß** das Teil in aufeinanderfolgenden Scheiben analysiert wird, wobei jede Scheibe in ringförmige Kränze unterteilt und jeder Kranz in Winkelsektoren unterteilt ist, wobei jeder Winkelsektor einen Analysebereich bildet.

7. Verfahren nach Anspruch 6, **dadurch gekennzeichnet, daß** jede Scheibe während einer Umdrehung des Teils analysiert wird.

8. Verfahren nach Anspruch 6, **dadurch gekennzeichnet, daß** die Analyse von zwei aufeinanderfolgenden Sektoren durch eine Umfangsbewegung des Teils um einen vorbestimmten und in etwa konstanten Vorschubschritt, unabhängig von der Tiefe des analysierten Bereichs, durchgeführt wird.

**Fig : 1**

**Fig : 2**

∅ efficace

Direction
tir US

Circonférence
billette

a

∅ efficace

<u>Fig : 3</u>

ω t

P

FE₁

FE₂

RT₁

RT₂

RT₃

θ

$$\theta\,(rd) = \frac{2\pi\,\omega}{F}$$

<u>Fig : 4</u>

Fig : 5

Fig : 6

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2698170